# EUROPÄISCHE PATENTSCHRIFT

(11) **EP 0 598 229 B1**
(45) Veröffentlichungstag und Bekanntmachung des Hinweises auf die Patenterteilung: **18.09.1996**
(21) Anmeldenummer: 93116792.8
(22) Anmeldetag: 18.10.1993
(51) Int. Cl.: C07C 45/52, C07C 47/22

(54) **Verfahren zur Herstellung von Acrolein**
Process for the preparation of acrolein
Procédé de préparation d'acroléine

(30) Priorität: 14.11.1992 DE 4238493
(43) Veröffentlichungstag der Anmeldung: 25.05.1994
(73) Patentinhaber: Degussa Aktiengesellschaft, D-60311 Frankfurt (DE)
(72) Erfinder: Neher, Armin, Dr., D-63636 Brachttal (DE); Haas, Thomas, Dr., D-60316 Frankfurt (DE); Arntz, Dietrich, Dr., D-61440 Oberursel (DE); Klenk, Herbert, Dr., D-63457 Hanau (DE); Girke, Walter, D-63457 Hanau (DE)

(56) Entgegenhaltungen:
- FR-A- 695 931
- US-A- 1 672 378
- US-A- 2 042 224
- US-A- 2 558 520

## Beschreibung

Die Erfindung betrifft ein Verfahren zur Herstellung von Acrolein oder wäßriger Acroleinlösung durch Dehydratisierung von Glycerin in der Flüssigphase oder Gasphase an sauren Feststoffkatalysatoren sowie eine Verwendung der wäßrigen Acroleinlösung.

Es ist bekannt, daß sich Glycerin in Gegenwart saurer Stoffe dehydratisieren läßt:

Gemäß Organic Synthesis I, 15-18 (1964) wird durch Behandeln eines Gemisches aus pulverförmigem Kaliumhydrogensulfat, Kaliumsulfat und Glycerin bei 190 bis 200 °C Acrolein in einer Ausbeute zwischen 33 und 48 % der Theorie gewonnen. Nachteilig an diesem Verfahren ist nicht nur die niedrige Ausbeute, sondern auch das erforderlich hohe Gewichtsverhältnis dehydratisierend wirkender Salze zu Glycerin. Dieses Verfahren eignet sich somit nicht für die Herstellung von Acrolein im technischen Maßstab.

Die Einsatzmenge an saurem Katalysator, wie Schwefelsäure, läßt sich niedrig halten, wenn die Dehydratisierung von Glycerin in homogener Phase oberhalb des kritischen Drucks von Wasser durchgeführt wird - siehe S. Ramayya et al. in FUEL (1987) Vol. 66, Oct., 1364-1371. Unter den in Tabelle 4 dieses Dokuments genannten Bedingungen - 34,5 MPa, 350 °C, 0,5 molare wäßrige Glycerinlösung - liegt der Umsatz an Glycerin bei 39 bis 55 %, wobei die Hauptprodukte Acrolein und Acetaldehyd im Gewichtsverhältnis von etwa 3:1 bis 4:1 entstehen. Der hohe technische Aufwand für das Arbeiten im überkritischen Bereich sowie die Rezyklierung beziehungsweise Entsorgung der Schwefelsäure macht das Verfahren wenig attraktiv für eine Acroleinproduktion im technischen Maßstab.

Untersucht wurde auch die Bildung von Acrolein aus Glycerin in der Gasphase, und zwar unter Bedingungen der Reaktionsgaschromatographie (Ishikawa Koichi et al., Bunseki Kagaku 32 (10) E 321 - E 325, zitiert in Chemical Abstracts 101 (4):32598w): Hierbei wird eine sehr verdünnte wäßrige Glycerinlösung (1,5 - 150 mg/l) bei 260 bis 300 °C in gepulster Form über eine mit 10 bis 30 % KHSO₄ beschichtete Gaschrom R-Säule geleitet. Ein Fachmann erhält aus diesem Dokument keine Anregung, das Analysenverfahren zur Grundlage eines technischen Verfahrens zur Herstellung von Acrolein aus Glycerin lassen zu machen, weil Glycerin nur äußerst verdünnt zur Anwendung kommt und die Säule durch die Pulsreaktion praktisch nicht belastet wird.

Aus der FR-PS 695 931 ist ein Verfahren zur Herstellung von Acrolein aus Glycerin bekannt, wobei Glycerindämpfe bei über 300 °C, inbesondere 400 bis 420 °C, über einen Festbettkatalysator geleitet werden. Als Katalysator werden Salze dreibasiger Säuren oder Mischungen solcher Salze, welche sich auf auf einem Träger befinden können, beansprucht; ausweislich der Beispiele wird mit 1 % Lithiumphosphat beziehungsweise 1 % Eisenphosphat belegter Bimsstein eingesetzt. In diesem Dokument wird die Acrolein-Ausbeute vorbekannter Verfahren in der Flüssigphase oder Gasphase unter Verwendung von KHSO₄ beziehungsweise MgSO₄ mit 20 beziehungsweise 30 % angegeben, jene des beanspruchten Verfahrens ausweislich der Beispiele mit 75 beziehungsweise 80 %.

Die Erfinder der vorliegenden Patentanmeldung haben das Verfahren der FR-PS 695,931 nachgearbeitet und dabei festgestellt, daß unter den getesteten Reaktionsbedingungen weder mit Lithiumphosphat noch mit Eisenphosphat die ausgewiesenen Ausbeuten erhalten werden konnten: Wie die Vergleichsbeispiele zeigen, lag bei 300 °C die Ausbeute an Acrolein nur bei etwa 1 bis 3 % und bei 400 °C bei 30 bis 35 %; in erheblichem Umfang wurden als Nebenprodukte Allylalkohol, Acetalaldehyd und Propionaldehyd gebildet. Nachteilig an dem Verfahren der FR-PS 695 931 ist somit die unbefriedigend niedrige Selektivität der Umsetzung und damit die niedrige Ausbeute an Acrolein.

Im Rahmen der Untersuchung von Modellsubstanzen von Biomasse-Pyrolyseölen wurde auch die katalytische Behandlung von Glycerin an H-ZSM5-Zeolithen bei 350 bis 500 °C untersucht - siehe Dao, Le H. et al. ACS Symp. Ser., 376 (Pyrolysis Oils Biomass), 328-341 (1988), zitiert in Chem. Abstracts 110 (6) : 41924n. Kohlenwasserstoffe werden nur in geringer Ausbeute gebildet, jedoch wird auf die Bildung von Acrolein hingewiesen. Wie die Erfinder der vorliegenden Patentanmeldung feststellten, ist die Selektivität der Dehydratisierung bei z. B. 380 °C in der Flüssigphase an H-ZSM5 wenig befriedigend. Hinzu kommt, und dies ist für ein technisches Verfahren von ausschlaggebender Bedeutung, daß unter den genannten Bedingungen die Standzeit des Katalysators auf wenige Stunden begrenzt ist.

Aufgabe der Erfindung ist, ein technisches Verfahren zur Herstellung von Acrolein oder wäßriger Acroleinlösung durch Dehydratisierung von Glycerin in Gegenwart von Heterogenkatalysatoren bereitzustellen, das sich mit hoher Raum-Zeit-Ausbeute und unter Vermeidung der Nachteile vorbekannter gattungsgemäßer Verfahren betreiben läßt. Gefordert werden ferner eine hohe Katalysatorstandzeit sowie eine hohe Selektivität.

Eine weitere Aufgabe richtet sich darauf, daß die beim Verfahren unmittelbar erhältliche wäßrige Acroleinlösung ohne weitere Aufarbeitung oder Reinigung der Verwendung zugeführt werden kann.

Gefunden wurde ein Verfahren zur Herstellung von Acrolein oder wäßriger Acroleinlösung durch Dehydratisierung von Glycerin, wobei ein Glycerin-Wasser-Gemisch entweder in der Flüssigphase oder in der Gasphase oberhalb 180 °C an einem Feststoffkatalysator umgesetzt und, sofern erforderlich, Acrolein oder wäßrige Acroleinlösung in bekannter Weise destillativ aus dem Reaktionsgemisch der Umsetzung abgetrennt wird, das dadurch gekennzeichnet ist, daß man ein Glycerin-Wasser-Gemisch mit einem Glyceringehalt von 10 bis 40 Gew.-% entweder in der Flüssigphase bei 180 bis 340 °C oder in der Gasphase bei 250 bis 340 °C an einem sauren Feststoffkatalysator umsetzt, wobei der Hₒ-Wert (Hammett'sche Säurefunktion) des Katalysators kleiner als +2 ist.

Erfindungswesentlich ist, daß wäßrige Glycerinlösung mit einer Konzentration zwischen 10 und 40 Gew.-%, vorzugsweise zwischen 10 und 25 Gew.-%, über den festen Dehydratisierungskatalysator geleitet wird. Zwar kommt es bei der Verwendung einer Glycerinlösung mit einem Gehalt oberhalb 40 Gew.-% noch zur Dehydratisierung, jedoch sinken mit steigender Glycerinkonzentration sowohl die Selektivität der Umsetzung als auch die Standzeit des Katalysators merklich ab. Die sinkende Selektivität macht sich insbesondere durch einen wachsenden Hochsiederanteil bemerkbar. Wäßriges Glycerin mit einer Konzentration unter 10 Gew.-% läßt sich zwar einsetzen, jedoch wird die Wirtschaftlichkeit des Verfahrens mit abnehmender Konzentration gemindert, da die Raum-Zeit-Ausbeute abnimmt und sowohl der Aufwand zur Konzentrierung des Acroleins als auch der Energieaufwand zur Verdampfung des Wassers zunimmt.

Das Verfahren läßt sich in der Flüssigphase oder in der Gasphase durchführen. In beiden Ausführungsformen können im Prinzip die gleichen sauren Feststoffkatalysatoren eingesetzt werden; es hat sich aber gezeigt, daß bestimmte Katalysatoren vorzugsweise für die Dehydratisierung in der Gasphase und andere, vorzugsweise für jene in der Flüssigphase, geeignet sind.

Die Umsetzung in der Gasphase wird besonders bevorzugt, weil der Glycerinumsatz praktisch 100 % beträgt und das den Katalysator verlassende gasförmige Reaktionsgemisch unter Erhalt einer wäßrigen Acroleinlösung, welche zusätzlich gebildete Nebenprodukte enthält, unmittelbar kondensiert werden kann; dieses Kondensat läßt sich vielfach unmittelbar weiterverarbeiten. Sofern erwünscht, kann aus dem Reaktionsgemisch Acrolein, gegebenenfalls gemeinsam mit einem Teil Wasser, durch Destillation oder fraktionierte Kondensation gewonnen werden.

Bei der Umsetzung in der Flüssigphase ist es zweckmäßig, die Dehydratisierung nur bis zu einem Glycerinumsatz von etwa 15 bis 25 % durchzuführen, da bei einer Steigerung des Umsatzes die Selektivität abnimmt. Nach Erreichen des genannten Umsatzes wird gebildetes Acrolein allein oder zusammen mit einem Teil des Wassers in bekannter Weise, üblicherweise destillativ oder durch eine N₂-Strippung aus dem Reaktionsgemisch abgetrennt. Das Acrolein kann durch Kondensation oder eine Wäsche mit Wasser isoliert werden. Das vom Acrolein befreite glycerinhaltige Reaktionsgemisch wird in die Dehydratisierungsstufe zurückgeführt. Ein Vorteil der Dehydratisierung in der Flüssigphase gegenüber jener in der Gasphase besteht in dem geringeren Energieaufwand, weil nur das aus dem Reaktionsgemisch abgetrennte Acrolein sowie ein damit übergehender Teil Wasser verdampft werden müssen.

Die Dehydratisierung in der Gasphase erfolgt vorzugsweise im Temperaturbereich zwischen 270 und 320 °C, jene in der Flüssigphase vorzugsweise zwischen 250 und 300 °C. Im Falle der Flüssigphasendehydratisierung wird die Apparatur mit mindestens einem solchen Druck beaufschlagt, der zur Aufrechterhaltung der Flüssigphase erforderlich ist.

Eine Steigerung der Temperatur auf über 340 °C bewirkt eine deutliche Abnahme der Selektivität. Überraschenderweise wurde gefunden, daß durch eine Begrenzung der Temperatur in der Dehydratisierungsstufe auf 340 °C und vorzugsweise die vorgenannten oberen Grenzwerte die Standzeit der Katalysatoren derart gesteigert wird, daß sie im Dauerbetrieb im technischen Maßstab eingesetzt werden können.

Als saure Feststoffkatalysatoren werden feste, im Reaktionsmedium im wesentlichen unlösliche ein- oder mehrphasig aufgebaute Stoffe mit einem Hₒ-Wert kleiner +2, vorzugsweise kleiner -3 eingesetzt. Der Hₒ-Wert entspricht der Säurefunktion nach Hammett und läßt sich durch die sogenannte Amintitration unter Verwendung von Indikationen oder durch Adsorption einer gasförmigen Base ermitteln - siehe Studies in surface science and catalysis, Vol. 51, 1989: "New solid acids and bases, their catalytic properties" by K. Tanabe et al. Kapitel 2, insbesondere Seiten 5-9. Kapitel 1 (Seiten 1-3) des vorgenannten Dokuments nennt zahlreiche feste Säuren, aus welchen der Fachmann, gegebenenfalls nach Bestimmung des Hₒ-Wertes, den geeigneten Katalysator auswählen kann. Als Heterogenkatalysatoren für die Dehydratisierung eignen sich vorzugsweise (i) natürliche und synthetische silikatische Stoffe, wie insbesondere Mordenit, Montmorillonit, saure Zeolithe; (ii) mit ein-, zwei- oder mehrbasigen anorganischen Säuren, insbesondere Phosphorsäure, oder sauren Salzen anorganischer Säuren belegte Trägerstoffe, wie oxidische oder silikatische Stoffe, beispielsweise Al₂O₃, TiO₂; (iii) Oxide und Mischoxide, wie beispielsweise gamma-Al₂O₃ und ZnO-Al₂O₃-Mischoxide oder Heteropolysäuren.

Für die Dehydratisierung in der Gasphase werden Katalysatoren mit einem Hₒ-Wert zwischen -3 und -8,2 besonders bevorzugt; hierzu zählt H₃PO₄ auf Al₂O₃ (-5,6 <Hₒ <-3), sogenannte feste Phosphorsäure.

Die Herstellung der Katalysatoren vom Typ (ii) ist besonders einfach, soweit sie in der Gasphasendehydratisierung zum Einsatz kommen. Das Trägermaterial wird mit einer wäßrigen Lösung der Säure behandelt und der so behandelte Feststoff getrocknet. Für den Fall der Flüssigphasendehydratisierung ist es empfehlenswert, an die Trocknung eine Temperung bei erhöhter Temperatur - beispielsweise 0,5 - 2 h bei 400 bis 500 °C - anzuschließen, um die Säure an der Trägeroberfläche zu fixieren.

Während Katalysatoren vom Typ Zeolith-H-ZSM5 wegen ihres Hₒ-Wertes von kleiner -8,2 für die Gasphasendehydratisierung weniger geeignet sind, sind sie bei der Flüssigphasendehydratisierung sogar bevorzugt.

Das erfindungsgemäße Verfahren kann in üblichen Apparaturen, wie sie dem Fachmann für Gasphasenbeziehungsweise Flüssigphasenreaktionen an einem Feststoffkatalysator geläufig sind, durchgeführt werden.

Die erfindungsgemäß erhältliche wäßrige Acroleinlösung kann unmittelbar der Verwendung, etwa der Herstellung von 1,3-Propandiol durch katalytische Hydratisierung zu 3-Hydroxypropionaldehyd mit nachfolgender katalytischer Hydrierung, zugeführt werden.

Ein besonderer Vorteil des erfindungsgemäßen Verfahrens besteht darin, daß wäßrige Glycerinlösungen mit einem Gehalt von 10 bis 40 Gew.-% verwendbar sind. Damit lassen sich sogenannte Rohglycerine direkt der Herstellung von Acrolein oder wäßriger Acroleinlösungen zuführen; eine vorhergehende Aufkonzentrierung und Reinigung des Rohglycerins entfällt damit.

Die einfache Verfahrensführung führt bei hoher Selektivität der Umsetzung zu einer hohen Raum-Zeit-Ausbeute. Die besondere Auswahl der Reaktionstemperatur und der Glycerinkonzentration, sowie die Zugabe hoher H₂O-Mengen, ermöglichen es, eine hohe Standzeit des Heterogenkatalysators zu erhalten. Anhand der nachfolgenden Beispiele und Vergleichsbeispiele wird die Erfindung weiter verdeutlicht.

### Beispiele 1 bis 3

### Dehydratisierung in der Gasphase:

### Beispiel 1

100 g Rosenthal-Kugeln (alpha-Al₂O₃) mit einem Durchmesser von 3 mm werden mit 25 g einer 20 gew.-%igen Phosphorsäure 1 h gemischt. Am Rotationsverdampfer wird bei 80 °C das überschüssige Wasser abgezogen. 100 ml dieses Katalysators (-5,6 <Hₒ <-3) werden in ein Stahlrohr von 15 mm Durchmesser eingefüllt. Es wird eine 20 gew.-%ige wäßrige Glycerin-Lösung über eine Pumpe mit 40 ml/h in einen auf 300 °C beheizten Verdampfer gefördert und dieser Gasstrom direkt über den Katalysator bei 300 °C geleitet. Bei quantitativem Glycerin-Umsatz erhält man eine Ausbeute von 70,5 % Acrolein in dem kondensierten Produktstrom. Als wesentliches Nebenprodukt sind ca. 10 %, bezogen auf Glycerin, 1-Hydroxyaceton enthalten.

Nach 60 h Betriebsdauer zeigt der Katalysator noch keinen Aktivitätsverlust.

### Beispiel 2a und 2b

a) Beispiel 1 wurde wiederholt, wobei jedoch 40 gew.-%ige wäßrige Glycerin-Lösung eingesetzt wurde. Die Ausbeute an Acrolein betrug 65 %.
b) Bei einer Acrolein-Anfangskonzentration von 10 Gew.-% betrug die Acrolein-Ausbeute 75 %.

### Beispiel 3

Beispiel 1 wurde wiederholt, wobei jedoch bei der Katalysatorherstellung 40 gew.-%ige Phosphorsäure eingesetzt wurde. Ausbeute an Acrolein 69,2 %.

### Beispiel 4

### Weiterverarbeitung der gemäß Beispiel 1 erhaltenen 8,6 gew.-%igen wäßrigen Acroleinlösung zu 1,3- und 1,2-Propandiol (PD):

Die gesamte Acroleinlösung wurde gemäß DE-A 40 38 192 an einem Ionenaustauscher mit Iminodiessigsäure-Ankergruppen (Lewatit TP 208, Fa. Bayer AG) umgesetzt, wobei die Hydratisierung zu 3-Hydroxypropionaldehyd erfolgt. Das nicht umgesetzte Acrolein wurde als 96 %iges Azeotrop mit H₂O destillativ vom Produktgemisch getrennt und mit dem Produktstrom der Glycerindehydratisierung vereinigt. Unter stationären Bedingungen erhielt man so eine Acroleinanfangskonzentration vom 14,3 %, Reaktionstemperatur 50 °C, LHSV = 0,5 h⁻¹, Umsatz = 60 %, Selektivität = 85 %. Die Produktlösung, 9,6 % an Hydroxypropionaldehyd, wurde gemäß DE-Patentanmeldung P 42 18 282.4 hydriert. Es wurde eine 9,9 gew.-%ige 1,3-Propandiol-Lösung erhalten. Neben den bekannten Nebenprodukten bei der Herstellung von 1,3 Propandiol aus Acrolein enthielt die Lösung noch einen Anteil von etwa 1 Gew.-% 1,2-Propandiol (1,2 PD). Dieses konnte aufgrund der Siedepunktdifferenz von 25 °C destillativ gut abgetrennt werden; Siedepunkte bei 50 mbar 109 °C für 1,2 PD und 134 °C für 1,3 PD. Das abdestillierte 1,3 PD hatte die gleiche Produktqualität wie ein 1,3 PD, ausgehend von einem Acrolein, das durch Propenoxidation gewonnen wurde. Die Ausbeute an 1,3 PD, bezogen auf eingesetztes Glycerin, betrug 60 %, jene an 1,2 PD 10 %.

### Beispiele 5 bis 7

### Dehydratisierung in der Flüssigphase

In einer Laborapparatur mit einem Festbettreaktor wird die Glycerindehydratisierung kontinuierlich über einen längeren Zeitraum durchgeführt. Umsatz und Selektivität werden durch Analyse der Produktlösung bestimmt. Die Apparatur besteht aus einer Vorlage für die Glycerin-Lösung, einer HPLC-Pumpe zur Förderung, einem Heißluftofen, in dem eine Vorheizstrecke sowie das Reaktionsrohr (160 x 15 mm i. D. ) installisiert sind. Nach dem Reaktor wird die Flüssigkeit auf Raumtemperatur gekühlt. Die gesamte Apparatur wird auf einem Druck von 70 bar gehalten, um das Verdampfen des Wassers zu vermeiden. Die Produktlösung wird in regelmäßigen zeitlichen Abständen analysiert.

| Beispiel Nr. | Katalysator | Temp. (°C) | Druck (bar) | LHSV (h⁻¹) | C_{Gly.O} (G%) | C_{AC} (G%) | U (%) | S (%) | Hₒ |
|---|---|---|---|---|---|---|---|---|---|
| 5 | Zeolith HZSM-5 Modul 60 | 250 | 70 | 2 | 10 | 0,74 | 16 | 75 | <-8,2 |
| 6 | Zeolith HZSM-5 Modul 28 | 300 | 70 | 2 | 10 | 0,8 | 19 | 71 | <-8,2 |
| 7 | Mordenit | 250 | 70 | 2 | 10 | 0,35 | 8 | 71 | <-8,2 |
| C_{Gly.O} = Einsatzkonzentration Glycerin C_{AC} = Einsatzkonzentration Acrolein U = Umsatz S = Selektivitat LHSV = liquid hourly space velocity | | | | | | | | | |

Die Katalysatoren zeigten nach 50 h Betriebsdauer noch keinerlei Aktivitätsabfall.

### Vergleichsbeispiele VB 1 bis VB 4

### VB 1:

### Flüssigphasendehydratisierung analog Beispiel 5, jedoch bei erhöhter Temperatur unter den nachfolgenden Bedingungen:

| Katalysator | Temp. (°C) | Druck (bar) | LHSV (h⁻¹) | C_{Gly.O} (G%) | C_{AC} (G%) | U (%) | S (%) |
|---|---|---|---|---|---|---|---|
| Zeolith HZSM-5 Modul 60 | 380 | 180 | 5 | 10 | 0,75 | 22 | 56 |

Der Katalysator war nach 6 h Betriebsdauer schwarz. Die Aktivität war nahezu auf 0 gesunken.

### VB 2:

### Gasphasendehydratisierung mit erhöhter Glycerinkonzentration:

Beispiel 1 wurde wiederholt, wobei jedoch 80 gew.-%iges Glycerin einsetzt wurde. Die Anfangsausbeute an Acrolein betrug nur 45 %; der Anteil an Hochsiedern stieg kräftig an. Bereits nach 4 h Betriebszeit war der Katalysator merklich desaktiviert.

### VB 3:

### Nacharbeitung des Verfahrens der FR-PS 695 931:

Als Trägermaterial dienten Tabletten aus pyrogener Kieselsäure. Die Tabletten wurden mit (a) 1 % Li₃PO₄ bzw. (b) mit 1 % FePO₄ belegt. Eingesetzt wurde 20 gew.-%iges Glycerin. Ergebnisse:

| | Katalysator | Temperatur | Ausbeute an Acrolein |
|---|---|---|---|
| (a) | Li₃PO₄ | 400 °C | 31,5 % |
| | | 300 °C | 1,3 % |
| (b) | FePO₄ | 400 °C | 35,7 % |
| | | 300 °C | 2,3 % |

Wesentliche Nebenprodukte waren unter anderem Allylalkohol und Acetaldehyd.

### VB 4:

### Gasphasendehydratisierung mit getempertem Katalysator:

Der Katalysator, hergestellt gemäß Beispiel 1, wurde 2 h bei 400 °C getempert. Anschließend wurde unter Verwendung dieses Katalysators unter sonst gleichen Bedingungen wie in Beispiel 1 dehydratisiert.

Die Anfangsausbeute an Acrolein betrug 36,6 %; die Ausbeute sank nach 4 h bereits auf 26,5 % ab.

### VB 5 und 6:

Die Flüssigphasendehydratisierung mit dem Katalysator Na-Zeolith A (VB 5), dessen Hₒ-Wert über 2,0 liegt, führt, wie aus der Tabelle folgt, zu praktisch keinem Umsatz.

Die Flüssigphasendehydratisierung mit gamma-Al₂O₃ als Katalysator (VB 6), dessen Hₒ-Wert größer 1,5 und kleiner 4,0 (1,5 <Hₒ <4,0) ist, führt zu einer völlig unzureichenden Selektivität, und zwar selbst dann, wenn die Glycerinausgangskonzentration sehr niedrig ist - siehe Tabelle.

| Nr. | Katalysator | Temp. (°C) | Druck (bar) | LHSV (h⁻¹) | C_{Gly.O} (G%) | C_{AC} (G%) | U (%) | S (%) |
|---|---|---|---|---|---|---|---|---|
| VB5 | Na-ZSM5 | 250 | 70 | 2 | 10 | - | <1 | - |
| VB6 | gamma-Al₂O₃ | 350 | 70 | 2 | 1 | 0,04 | 22 | 30 |

## Patentansprüche

1. Verfahren zur Herstellung von Acrolein oder wäßriger Acroleinlösung durch Dehydratisierung von Glycerin, wobei ein Glycerin-Wasser-Gemisch entweder in der Flüssigphase oder in der Gasphase oberhalb 180 °C an einem Feststoffkatalysator umgesetzt und, sofern erforderlich, Acrolein oder wäßrige Acroleinlösung in bekannter Weise destillativ aus dem Reaktionsgemisch der Umsetzung abgetrennt wird,
dadurch gekennzeichnet,
daß man ein Glycerin-Wasser-Gemisch mit einem Glyceringehalt von 10 bis 40 Gew.-% entweder in der Flüssigphase bei 180 bis 340 °C oder in der Gasphase bei 250 bis 340 °C an einem sauren Feststoffkatalysator umsetzt, wobei der Hₒ-Wert (Hammett'sche Säurefunktion) des Katalysators kleiner als +2 ist.

2. Verfahren nach Anspruch 1,
dadurch gekennzeichnet,
daß man die Umsetzung in der Gasphase bei 270 bis 320 °C durchführt.

3. Verfahren nach Anspruch 1,
dadurch gekennzeichnet,
daß man die Umsetzung in der Flüssigphase bei 250 bis 300 °C und mindestens dem Druck, der zur Aufrechterhaltung der Flüssigphase notwendig ist, durchführt.

4. Verfahren nach Anspruch 3,
dadurch gekennzeichnet,
daß man die Dehydratisierung bis zu einem Glycerin-Umsatz von 15 bis 25 % führt, gebildetes Acrolein allein oder zusammen mit einem Teil des Wassers durch Strippen aus dem Reaktionsgemisch abtrennt und das acroleinfreie Reaktionsgemisch in die Dehydratisierungsstufe zurückführt.

5. Verfahren nach Anspruch 1 oder 2,
dadurch gekennzeichnet,
daß man bei der Umsetzung in der Gasphase einen sauren Feststoffkatalysator mit einem Hₒ-Wert zwischen +2 und -8,2 , vorzugsweise zwischen -3 und -8,2 , verwendet.

6. Verfahren nach einem oder mehreren der Ansprüche 1, 3 oder 4,
dadurch gekennzeichnet,
daß man als Katalysator bei der Umsetzung in der Flüssigphase einen Zeolith vom H-ZSM5- oder H-Y-Typ verwendet.

## Claims

1. Process for the production of acrolein or aqueous acrolein solution by dehydration of glycerol, wherein a glycerol-water mixture is reacted either in the liquid phase or in the gas phase at above 180°C on a solid catalyst and, if necessary, acrolein or aqueous acrolein solution is separated in a known manner by distillation from the reaction mixture,
characterised in that
a glycerol-water mixture with a glycerol content of 10 to 40 wt.% is reacted on an acidic solid catalyst either in the liquid phase at 180 to 340°C or in the gas phase at 250 to 340°C, wherein the Hₒ value (Hammett acidity function) of the catalyst is less than +2.

2. Process according to claim 1,
characterised in that
the reaction is performed in the gas phase at 270 to 320°C.

3. Process according to claim 1,
characterised in that
the reaction is performed in the liquid phase at 250 to 300°C and at at least the pressure necessary to maintain the liquid phase.

4. Process according to claim 3,
characterised in that
dehydration is continued to a glycerol conversion of 15 to 25%, the acrolein formed is separated from the reaction mixture alone or with a proportion of the water by stripping and the acrolein-free reaction mixture is returned to the dehydration stage.

5. Process according to claim 1 or 2,
characterised in that,
in the gas phase reaction, an acidic solid catalyst is used with an Hₒ value of between +2 and -8.2, preferably between -3 and -8.2.

6. Process according to one or more of claims 1, 3 or 4,
characterised in that,
in the liquid phase reaction, a zeolite of the H-ZSM5 or H-Y type is used as the catalyst.

## Revendications

1. Procédé de préparation d'acroléine ou d'une solution d'acroléine aqueuse par déshydratation de glycérine, dans lequel on fait réagir un mélange de glycérine et d'eau en phase liquide ou en phase gazeuse au'dessus de 180°C sur un catalyseur solide et, pour autant que cela soit nécessaire, on sépare l'acroléine ou la solution d'acroléine aqueuse de manière connue par distillation du mélange réactionnel,
caractérisé en ce qu'on fait réagir un mélange de glycérine et d'eau ayant une teneur en glycérine de 10 à 40 % en poids, respectivement à 180 à 340°C en phase liquide ou à 250 à 340°C en phase gazeuse, sur un catalyseur solide acide, la valeur de Hₒ (fonction acide de Hammett) du catalyseur étant inférieure à +2.

2. Procédé selon la revendication 1,
caractérisé en ce que l'on effectue la réaction en phase gazeuse à 270 à 320°C.

3. Procédé selon la revendication 1,
caractérisé en ce que la réaction en phase liquide est réalisée á 250 á 300°C et au moins à la pression qui est nécessaire pour maintenir la phase liquide.

4. Procédé selon la revendication 3,
caractérisé en ce qu'on contrôle la déshydratation jusqu'à une réaction de la glycérine de 15 à 25 %, on sépare du mélange réactionnel par extraction l'acroléine formée seule ou conjointement avec une partie de l'eau, et on renvoie le mélange réactionnel exempt d'acroléine au stade de déshydratation.

5. Procédé selon la revendication 1 ou 2,
caractérisé en ce que l'on utilise pour la réaction en phase gazeuse un catalyseur solide acide ayant une valeur de Hₒ comprise entre +2 et -8,2, de préférence entre -3 et -8,2.

6. Procédé selon une ou plusieurs des revendications 1, 3 ou 4,
caractérisé en ce qu'on utilise comme catalyseur dans la réaction en phase liquide une zéolite de type H-ZSM5 ou H-Y.
